# EUROPEAN PATENT SPECIFICATION

(11) **EP 0 287 397 B1**
(45) Date of publication and mention of the grant of the patent: **10.08.1994**
(21) Application number: 88303435.7
(22) Date of filing: 15.04.1988
(51) Int. Cl.: C12P 21/00, C12N 5/00, C12N 15/00, G01N 33/573, G01N 33/577

(54) **Monoclonal antibody specific to human pancreatic phospholipase A2**
Monoklonaler Antikörper spezifisch gegen humane Pankreas-Phospholipase-A2
Anticorps monoclonal spécifique contre la phospholipase A2 pancréatique humaine

(30) Priority: 15.04.1987 JP 93586/87
(43) Date of publication of application: 19.10.1988
(73) Proprietor: SHIONOGI SEIYAKU KABUSHIKI KAISHA trading under the name of SHIONOGI & CO. LTD., Osaka 541 (JP)
(72) Inventor: Misaki, Atsushi, Osaka-shi, Osaka (JP); Ogawa, Michio, Toyonaka-shi, Osaka (JP); Kono, Masao, Ibaraki-shi, Osaka (JP); Okamoto, Mitsuhiro, Ikoma-shi, Nara (JP)
(74) Representative: Allard, Susan Joyce

(56) References cited:
- BIOLOGICAL ABSTRACTS/RMM, vol. 33, abstract no. 101361, Philadelphia, PA, US; S.R. LANGTON et al.: "A highly sensitive assay for serum phospholipase A-2 PLA-2 using a monoclonal antibody"
- BIOLOGICAL ABSTRACTS/RMM, vol. 28, abstract no. 28108714, Philadelphia, PA,US; A. LUCAS et al.: "Analysis of human antibodies specific for the phospholipase A-2 of honey bee venom"
- J. BIOCHEM., vol. 94, no. 1, 1983, pages 137-147; J. NISHIJIMA et al.:"Purification and characterization of human pancreatic phospholipase A2 and development of a radioimmunoassay"
- CHEMICAL ABSTRACTS, vol. 106, no. 9, 2nd March 1987, page 262, abstract no.63383e, Columbus, Ohio, US; J.U. ESKOLA et al.: "Pancreatic phospholipase A2 in human acute pancreatis"
- CHEMICAL ABSTRACTS, vol. 106, May 1987, page 306, abstract no. 171628x,Columbus, Ohio, US; J. DE JONG et al.: "Monoclonal antibodies against an intracellular phospholipase A2 from rat liver and their cross-reactivity with other phospholipases A2"
- ISCU Short Reports, vol. 6, Contemporary Themes in Biochemistry, 4th Federation of Asian Oceanian Biochemists Congress, 30/12/1986, pages 108-109, Singapore, Cambridge University Congress

## Description

This invention relates to a monoclonal antibody specific to human pancreatic phospholipase A₂ and having other specific characteristics, its production, hybridomas producing it and an assay of human pancreatic phospholipase A₂ using it.

Phospholipase A₂ (hereinafter referred to as PLA₂) is an enzyme hydrolyzing diacylglycero- phospholipid in ester linkage at the second position. PLA₂ is roughly classified as intracellular or membraneous PLA₂ and PLA₂ secreted from pancreas as digestive enzyme. The latter is produced as prophospholipase A₂ in pancreatic acinar cells and is secreted into the pancreatic duct. Then it is hydrolyzed mainly by trypsin in the duodenum losing the amino terminal peptide chain comprising 7 amino acid residues to give active PLA₂. Up to now, isolation of human pancreatic PLA₂ from pancreas and pancreatic juice has been reported (Magee, W. L. et al, Biochem. J. 83, 17-25, 1962; Grataroli, R. et al, Biochimie 63, 677-684, 1981; Nishijima, J. et al, J. biochem., 94, 137-147, 1983 and so on).

It has been reported that in pancreatitis the blood level of pancreatic PLA₂ is elevated. Accordingly, an assay of pancreatic PLA₂ can be utilized in diagnosing acute abdomen conditions including acute pancreatitis.

Hitherto, pancreatic PLA₂ in serum has been assayed enzymatically. However, enzymatic activity of pancreatic PLA₂ in serum is low and, therefore, the assay requires a long and complicated procedure, which makes it practically impossible to clinically apply the assay for the diagnosis of acute abdomen conditions.

A radioimmunoassay of pancreatic PLA₂ by Nishijima et al (J. Biochem 94, 137-147, 1983) and fluorimmunoassay by Eskola et al (clin. Chem. 29, 1777, 1983) have already been reported. According to both methods, blood levels of PLA₂ in acute pancreatitis are higher than normal levels. This fact suggests that the immunoassay can be applied for diagnosing acute abdomen conditions including acute pancreatitis.

As described above, human pancreatic PLA₂ has been able to be assayed immunologically in some instances. However, the antibodies used in the above reports are polyclonal antibodies (rabbit antiserum). Polyclonal antibodies are inferior to monoclonal antibodies in terms of specificity, equality and stable supply. Moreover, if a monoclonal antibody having high affinity is obtained, it becomes possible to develop simpler and quicker assay methods for human pancreatic PLA₂.

ICSU, Short Reports, 1986, Vol. 6, pp. 108-109. Contemporary themes in biochemistry; 4th Federation of Asian Oceanian Biochemists Congress, Singapore, Cambridge University Press discloses a monoclonal antibody (mAb) specific to human pancreatic phospholipase A₂, which is of subclass IgG1. A hybridoma producing said mAb and a process for the production of said hybridoma producing said mAb are also disclosed, as well as an immunoassay for human pancreatic phospholipase A₂ using this mAb.

The present invention provides for a monoclonal antibody specific to human pancreatic phospholipase A₂ which is of subclass IgG1₁, has an L chain of the κ type and has the following characteristics:
a) an affinity constant greater than 10¹¹ M⁻¹;
b) does not react with porcine pancreatic phospholipase A₂ or Naja venom phospholipase A₂;
a method for their production, hybridomas producing them and the immunoassay for human pancreatic PLA₂ using them. The invention also includes kits for use in such assays in standard form and incorporating the said monoclonal antibodies. The hybridomas of this invention which produce anti-human pancreatic PLA₂ monoclonal antibody are thoroughly cloned cell lines. By culturing them the monoclonal antibodies described above are effectively manufactured. By using the monoclonal antibodies in an immunoassay such as a radioimmunoassay and the like, the quick diagnosis of acute abdomen conditions including acute pancreatitis becomes possible.

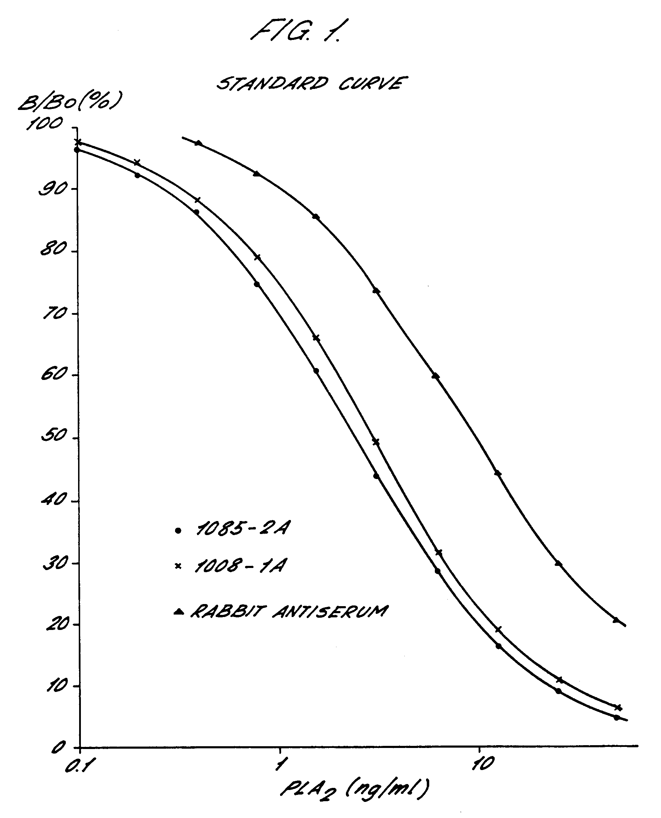

Fig. 1 shows the standard curve of pancreatic PLA₂ in radioimmunoassay using monoclonal or polyclonal antibody.

This invention provides a monoclonal antibody having high affinity and specificity for human pancreatic PLA₂ as described above and an assay method thereby which is effective for diagnosis of acute pancreatitis and so on.

Taking the above situation into consideration, the present inventors obtained a monoclonal antibody having high affinity and specificity.

The anti-human pancreatic PLA₂ monoclonal antibody of this invention is produced by a hybridoma which is prepared by fusing a myeloma cell with a lymphocyte obtained from human pancreatic PLA₂-immunized mice.

In order to obtain the monoclonal antibody of this invention, it is necessary to purify human pancreatic PLA₂ as an immunogen. That purification can be achieved according to the methods as described in the above mentioned reports.

A laboratory animal such as a mouse is immunized with the refined human pancreatic PLA₂ as immunogen. The immunization may be effected in a usual manner, for example, as an emulsion prepared by mixing the antigen in physiological saline with Freund's complete adjuvant which is injected.

B lymphocyte is prepared from the animal immunized according to the above manner and fused with a permanent myeloma cell line. This fusion may be performed according to the usual method (Köhler and Milstein, Nature 256, 495-497, 1975). From the obtained hybridomas, a hybridoma producing anti-human pancreatic PLA₂ antibody is selected.

This selection is achieved by assaying anti-human pancreatic PLA₂ antibodies in culture supernatant by suitable methods such as enzyme linked immunosorbent assay (ELISA) and radioimmunoassay. Hybridomas in the supernatant of which anti-human pancreatic PLA₂ antibody is detected are multiplied in about 10 ml of medium and frozen for preservation. At the same time the supernatant is recovered and the properties of antibody in the supernatant are examined to select a hybridoma producing the monoclonal antibody of desired properties. The selected hybridoma is cloned using usual methods such as limiting dilution to establish the cell line. The established cell line is transplanted into the abdominal cavity of the same species of animal as used in the immunization to obtain the ascites containing a high concentration of the antibody or cultured in a medium to obtain the antibody from the cultured medium.

Thus prepared antibody may be refined as occasion demands, for example, by the usual method such as ammonium sulfate-fractionation, ion-exchange chromatography, protein A column chromatography and the like.

In this invention, anti-human pancreatic PLA₂ antibody-producing mouse hybridoma cell lines 1008-1 and 1085-2 and monoclonal antibodies 1008-1 and 1085-2 produced thereby were obtained according to the above method.

Thus obtained hybridoma cell lines have been deposited under the following accession numbers with European Collection of Animal Cell Cultures (ECACC) at PHLS CAMR, Porton Down, Salisbury, SP4 OJG., Great Britain under the Budapest Treaty since April 8, 1987.
Hybridoma 1008-1 : 87040803
Hybridoma 1085-2 : 87040802
Monoclonal antibodies 1008-1 and 1085-2 have the following properties.

They are
(1) Immunoglobulins, of subclass IgG₁ having a κ type L chain.
(2) The binding constants of 1008-1 and 1085-2 with human PLA₂ are 4 x 10¹¹ M⁻¹ and 1 x 10¹² M⁻¹, respectively. Both have high affinity.
(3) Both monoclonal antibodies have no cross reactivity with porcine pancreatic PLA₂ and Naja naja venom (Indian cobra venom) PLA₂ and are specific to human pancreatic PLA₂.

Thus, the monoclonal antibodies of this invention are specific to human pancreatic PLA₂ and show very high affinity thereto. Therefore, they are very useful in the immunoassay of human pancreatic PLA₂.

When PLA₂ in human serum is measured by the radioimmunoassay method, utilizing ascites 1008-1A or 1085-2A, 1008-1A gives the PLA₂ concentration as 2.6 ± 0.7 ng/ml in normal adults and 44.1 ± 34.0 ng/ml in acute pancreatitis patients and 1085-2A gives, 0.8 ± 0.2 ng/ml and 23.8 ± 14.7 ng/ml. In both cases the latter is clearly higher than the former.

The result shows that the immunoassay such as radioimmunoassay and enzyme immunoassay using the monoclonal antibody of this invention can be applied to the diagnosis of acute pancreatitis. Moreover, by utilizing high affinity of the monoclonal antibodies of this invention a quick and simple immunoassay for human pancreatic PLA₂ can be developed and utilized in the diagnosis of acute pancreatitis.

It is not hard for the skilled man to prepare hybridomas and monoclonal antibodies having the properties similar to those of this invention. Such hybridomas and monoclonal antibodies having the properties specified in the claims are included in the scope of the invention.

### Example

### Example 1 : Preparation of hybridoma producing anti-human pancreatic PLA₂ monoclonal antibody and production of the antibody

### (1) Preparation of Antigen

Human pancreatic PLA₂ for use as an antigen was purified from human pancreatic juice according to the method of Nishijima et al (J. Biochemistry 94, 137-147, 1983).

### (2) Immunization

Human pancreatic PLA₂ in physiological saline (PLA₂ concentration 0.5 mg/ml) was emulsified with the same volume of Freund's complete adjuvant, 0.1 ml of which was subcutaneously injected into the back of BALB/c mouse (female, 5 weeks-old) 3 times at intervals of 3 weeks. The dose of PLA₂ was 25 µg per mouse. 5 weeks after the third immunization 50 µg of human pancreatic PLA₂ dissolved in 200 µl of physiological saline was injected into the abdominal cavity as booster.

### (3) Fusion

Four days after the booster, the spleen of the mouse was enucleated and the cells were kept in 0.17 M ammonium chloride with ice-cooling for 5 min to destroy the erythrocytes. The remaining cells were suspended in RPMI 1640 medium to prepare spleen lymphocytes to be used in cell-fusion. Next, 8-azaguanine-resistant myeloma cells (NS-1, 7 x 10⁷ cells) suspended in RPMI 1640 were mixed with the spleen cells (1.4 x 10⁸ cells), which were centrifuged at 1000 rpm for 10 min. After the supernatant was removed, 0.8 ml of 50% polyethyleneglycol (molecular weight 4000, Merck) dissolved in RPMI 1640 was added to the cell pellet over 1 min, with stirring, with a pippette and further stirred for 1.5 min. Then, 2 ml of RPMI 1640 was added thereto over 2 min with stirring and an additional 2 ml over 1 min. Further, 18 ml of RPMI 1640 was added dropwise thereto with gentle stirring. After centrifugation (1000 rpm, 10 min), the supernatant was removed and the cells sedimented were suspended in 70 ml of HAT medium (RPMI 1640 containing 20% FCS, 1 x 10⁻⁴ M hypoxanthine, 4 x 10⁻⁷ M aminopterin, 1.6 x 10⁻⁵ M thymidine), 0.1 ml of which was distributed to each well of 7 96-well cell culture plates (Coaster). In the cell culture plates mouse spleen cells suspended in HAT medium (1 x 10⁵ cells/0.1 ml/well) were previously placed as feeder cells. After that, half the amount of the HAT medium was replaced by fresh medium at intervals of a few days. About 10 days later, growth of hybridomas was observed. The number of wells in which hybridomas grew was 508 (75%).

### (4) Selection of hybridoma

It was determined by ELISA if anti-human pancreatic PLA₂ antibody was produced in the well in which hybridomas grew, using the supernatant. To each well of 96-well flat-bottom ELISA plates (Nunc) was added 50 µl of human pancreatic PLA₂ solution (10 ng/50 µl of 0.05 M sodium carbonate buffer, pH 8.5) and allowed to stand overnight at 4 °C to immobilize the human pancreatic PLA₂ to the plate. After removing the supernatant, 100 µl of PBS containing 0.2% bovine serum albumin was added thereto and allowed to stand at room temperature for 1 hr. Each well was washed twice with PBS containing 0.1 % Tween 80, to which was added about 50 µl of the supernatant from the well in which the hybridoma grew. This was allowed to stand at 37°C for 30 min. After washing these wells twice, 50 µl of biotinylated anti-mouse immunoglobulin antibody (Vectastain ABC kit, Funakoshi Yakuhin Kabusikigaisha) was added to each well and allowed to stand at room temperature for 15 min. After washing twice, 50 µl of a solution of complex of avidin with biotinylated horseradish peroxidase was added thereto and allowed to stand at room temperature for 15 min. These wells were washed 5 times, to which was added 100 µl of a substrate solution (0.05 M citrate-phosphate buffer (pH 5.3) containing 0.02 % ABTS and 0.03 % hydrogen peroxide) and allowed to stand at room temperature for 20 min. Finally, 100 µl of 2 mM sodium azide was added to each well and stirred and the absorbance at 415 nm was measured by a plate reader (MTP-22, Corona Electric Co. Ltd.).

### (5) Freezing of hybridoma for preservation

Those hybridomas which demonstrated activity as anti-human pancreatic PLA₂ antibody in their wells using the above ELISA were expanded into about 10 ml of HT medium (aminopterin was removed from the HAT medium described above.) and were suspended in 1 ml of the medium for freezing (bovine serum albumin containing 10% DMSO) and frozen at -80 °C for preservation. Each supernatant was recovered and preserved in order to examine the property of the produced antibody. Eighty five kinds of hybridomas in all were preserved.

### (6) Displacement test of supernatant

Serial dilution (100 µ l) of the supernatants which were recovered in preserving hybridomas as mentioned above were mixed with 100 µ l of ¹²⁵I-labelled human pancreatic PLA₂ (described in the example 5 (1)) and the assay buffer (described in the example 5 (1)) containing 2% bovine gamma globulin and incubated at room temperature overnight. Then, 250 µl of an aqueous solution of 27% polyethyleneglycol 6000 was added thereto and immediately stirred. After centrifugation at 3000 rpm for 20 min, the supernatant was removed by suction. The radioactivity of the precipitate was measured to calculate the ratio of ¹²⁵I-labelled human pancreatic PLA₂ bound to the antibody. At the same time, the same test was performed using 100 µ l of the assay buffer containing 5 ng/100 µ l of human pancreatic PLA₂ and 2% bovine gamma globulin instead of the assay buffer containing 2% bovine gamma globulin and the decrease of the radioactivity of ¹²⁵I-labelled human pancreatic PLA₂ from the precipitate, that is, the extent of displacement of ¹²⁵I-labelled human pancreatic PLA₂ by the supplemented human pancreatic PLA₂ was examined. The supernatant in which the ratio of the displacement was high was regarded as containing high affinity antibody.

### (7) Cloning

Hybridomas which were regarded as producing high affinity antibody by the result of the above displacement test were reconstituted from frozen samples and cloned by limiting dilution. In 96-well cell culture plates the hybridomas were cultured at a concentration of 1 cell/200 µ l/well. The ELISA described above was performed on the supernatant of the well in which hybridomas grew as a sole colony. The hybridomas of the wells in which the activity of anti-human pancreatic PLA₂ antibody was observed were expanded by clture. Thus, the hybridoma cell lines 1008-1 and 1085-2 which produce anti-human pancreatic PLA₂ antibody were established.

### (8) Preparation of ascites containing antibody

The established hybridomas were transplanted into the abdominal cavities of mice in order to prepare ascites containing antibody at a high concentration. Into a mouse (BALB/c, female, into which 0.5 ml of pristane was intraperitoneally injected 10 days before) was intraperitoneally injected about 1 x 10⁷ hybridomas suspended in RPMI 1640. The ascites were recovered 1 - 3 weeks later and the cells in the ascites were removed therefrom. To the supernatant was added sodium 0.1% azide and the resultant suspension was frozen for preservation. Thus, the ascites 1008-1A and 1085-2A containing the monoclonal antibodies at a high concentration, which were produced by the hybridomas 1008-1 and 1085-2, respectively, were prepared.

### (9) Purification of monoclonal antibody

Antibodies were purified from the ascites 1008-1A and 1085-2A by Affi-Gel Protein A MAPS-II kit (BIO-RAD). Using 2 ml of the gel, 1 ml of each ascites was purified according to the procedure of the kit and about 2 mg and about 5 mg of antibodies were obtained from 1008-1A and 1085-2A, respectively. The purity of the antibody was examined by SDS-polyacrylamide electrophoresis. The purified antibody fraction was reduced with 2-mercaptoethanol and applied to 12.5% SDS electrophoresis. As a result, 2 bands, one H chain around molecular weight about 52000 and one L chain around molecular weight about 28000 were observed.

### Example 2 : Determination of class and subclass of monoclonal antibody

The determination of class and subclass of immunoglobulin produced by the hybridoma was achieved by the ELISA described above, using rabbit antibody specific to class and subclass of mouse immunoglobulin and peroxidase-conjugated goat anti-rabbit antibody (MonoAb-ID EIA Kit, Zymed Laboratories). As a result, in both the cases of the ascites 1008-1A and 1085-2A development of color was observed when anti-γ ₁ antibody and anti-κ antibody were used.

### Example 3 : Affinity of monoclonal antibody

According to the procedure of the example 5 (2), a standard curve was prepared and the radioactivity of precipitate/radioactivity of supernatant and concentration of antigen bound to antibody were plotted in ordinate and abscissa respectively, against each concentration of human pancreatic PLA₂ according to the scatchard method. The affinity constant of the antibody was calculated from the slope of the resulting line.

### Example 4 : Specificity of monoclonal antibody

By using porcine pancreatic PLA₂ (Boehringer Mannheim GmbH) and Naja naja venom PLA₂ (Sigma) instead of a sample for the radioimmunoassay of human pancreatic PLA₂ in the example 5 (2), the displacement of ¹²⁵I-labelled human pancreatic PLA₂ was examined. As a result, in both cases of 1008-1A and 1085-2A, the displacement of ¹²⁵I-labelled human pancreatic PLA₂ was not observed at all where 2 kinds of PLA₂ described above were added at a thousand times as much as 50% inhibitory concentration in case of human pancreatic PLA₂.

### Example 5 : Radioimmunoassay using monoclonal antibody

### (1) Preparation of ¹²⁵I-labelled human pancreatic PLA₂

¹²⁵I-labelled human pancreatic PLA₂ was prepared by the chloramine-T method according to the usual method of Hunter and Greenwood (Nature 194, 495-496, 1962).

### Reagent :

Human pancreatic PLA₂ : 0.5 mg/ml 0.5 M phosphate buffer, pH 7.5
Na¹²⁵I : 1 mCi/10 µ l (Amersham)
Chloramine-T : 2 mg/ml 0.5 M phosphate buffer, pH 7.5
Sodium pyrosulfite : 2.5 mg/ml 0.5 M phosphate buffer, pH 7.4
Bovine serum albumin : 10 mg/ml 0.1 M phosphate buffer, pH 7.4
KI : 100 mg/ml distilled water

### Assay buffer :

0.01 M phosphate buffer (pH 7.4) containing 0.2% bovine serum albumin, 5 mM ethylenediamine tetraacetate, 0.01% sodium azide and 0.9% NaCl

### Freeze-drying buffer :

5 times the concentration of the assay buffer

### Method :

To a tube 25 µl of 0.5 M phosphate buffer (pH 7.5), 10 µl of human pancreatic PLA₂ (5 µg) and 5 µl of Na¹²⁵I (500 µCi) were added and stirred. To the resultant was added 5 µl of chloramine-T (10 µg) and stirred for 45 sec, to which was added 25 µl (62.5 µg) of sodium pyrosulfite and stirred. Then, 5 µl (50 µg) of bovine serum albumin and 5 µl (500 µg) of KI were added thereto and stirred. The reaction mixture was applied to a Sephadex G-25 column (1 cm diameter x 25 cm length) to separate ¹²⁵I-labelled human pancreatic PLA₂ and ¹²⁵I⁻ ion. The fraction of ¹²⁵I-labelled human pancreatic PLA₂ was diluted to 2.5 µCi/2 ml with freeze-drying buffer, 2 ml of which was distributed to a vial, freeze-dried and preserved at 4 °C. Before use, it was dissolved in 10 ml of distilled water for dissolving.

### (2) Radioimmunoasay of human pancreatic PLA₂

### Reagent :

Dilution of ascites : 1008-1A (diluted 360000 times with assay buffer) and 1085-2A (diluted 800000 times with assay buffer)
Human pancreatic PLA₂ standard : Assay buffer containing human pancreatic PLA₂ at a concentration of twofold dilution in a range of 0.1 - 25 ng/ml
¹²⁵I-labelled human pancreatic PLA₂ : described in (1)
Assay buffer : described in (1)
Immunobeads : Rabbit anti-mouse immunoglobulin (BIO-RAD), used as a 1 mg/ml solution diluted with assay buffer

### Method :

In a tube was placed 100 µl of human pancreatic PLA₂ standard or a sample to which 200 µl of assay buffer, 100 µl of ¹²⁵I-labelled human pancreatic PLA₂ and 100 µl of diluted ascites (1008-1A or 1085-2A) were added. After mixing, it was incubated overnight at room temperature. Then, 100 µl of immunobeads was added thereto and allowed to stand at room temperature for 2 hr. After centrifuging at 3000 rpm for 10 min and removing the supernatant, the radioactivity of the precipitate was measured by a gamma counter (Aloka, ARC-600). From the standard curve made by using human pancreatic PLA₂ standard the concentration of human pancreatic PLA₂ in the sample was calculated.

### Standard Curve

The standard curve of radioimmunoassay is shown in Fig. 1. The sensitivity (90% inhibitory concentration) in cases using 1008-1A and 1085-2A was 0.4 ng/ml and 0.3 ng/ml, respectively, which indicates the curve is highly sensitive.

### Assay of human serum

Sera of normal adults, patients with acute pancreatitis and patients from whom the whole pancreas is enucleated, which were used undiluted or diluted with the assay buffer to be used, were assayed by the radioimmunoassay. The results are shown in Table 1.

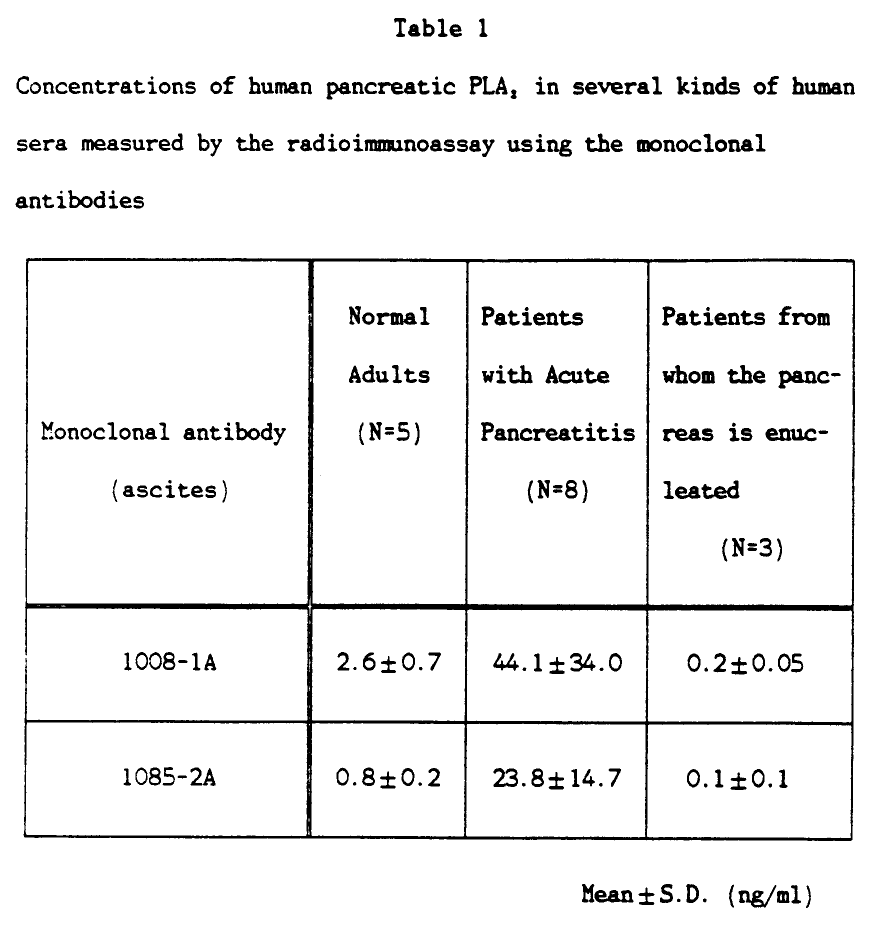

The concentration in serum of patients with acute pancreatitis was higher than that of normal adults in both radioimmunoassays using 1008-1A and 1085-2A. Thus, these 2 monoclonal antibodies were proved to be useful in the diagnosis of acute pancreatitis.

Since the concentrations found in patients from whom the whole pancreas was enucleated were under the detection limit, these monoclonal antibodies were proved to be highly specific to human pancreatic PLA₂.
Referential examination : Radioimmunoassay using rabbit antiserum
Standard curve was made by using rabbit antiserum in a similar manner to the example 5 except for the followings.
Diluted ascites → Diluted antiserum : diluted 40000 times with the assay buffer
Human pancreatic PLA₂ standard : Assay buffer containing human pancreatic PLA₂ at a concentration of twofold dilution in a range of 0.39 - 100 ng/ml
Immunobeads : Goat anti-rabbit immunoglobulin (BIO-RAD), diluted with the assay buffer to 1 mg/ml
The result is shown in Fig. 1. The 90% inhibitory concentration of the standard curve by rabbit antiserum was 1.0 ng/ml. Those of 1008-1A and 1085-2A are 0.4 ng/ml and 0.3 ng/ml, respectively, and, therefore, the monoclonal aitibodies 1008-1A and 1085-2A, are more sensitive than the rabbit antiserum.

## Claims

1. A monoclonal antibody specific to human pancreatic phospholipase A₂ which is of subclass IgG1₁, has an L chain of the κ type and has the following characteristics:-
a) an affinity constant greater than 10¹¹ M⁻¹;
b) does not react with porcine pancreatic phospholipase A₂ or Naja naja venom phospholipase A₂.

2. A hybridoma capable of producing a monoclonal antibody as claimed in claim 1.

3. A hybridoma capable of producing a monoclonal antibody specific to human pancreatic phospholipase A₂ or a monoclonal antibody specific to human pancreatic phospholipase A₂, in either case obtainable from ECACC 87040802 or ECACC 87040803.

4. A process either a) for the production of a hybridoma capable of producing a monoclonal antibody as defined in claim 1 or b) for the production of the said antibody, comprising fusing anti-human pancreatic phospholipase A₂ antibody producing cells with myeloma cells and, if desired, culturing the hybridoma to permit antibody production.

5. An immunoassay, preferably a radioimmunoassay, for human pancreatic phospholipase A₂ using a monoclonal antibody as defined in claim 1 as an immunoreagent.

6. A method of in vitro diagnosis or monitoring of abdominal condition, comprising assaying human pancreatic phospholipase A₂ employing an immunoassay as defined in claim 5.

7. An immunoassay kit comprising a monoclonal antibody as defined in claim 1, instructions for carrying out an immunoassay as defined in claim 5, and optionally a label permitting subsequent detection of a complex comprising said antibody and human pancreatic phospholipase A₂.

## Patentansprüche

1. Monoklonaler Antikörper, spezifisch gegen humane Pankreasphospholipase A₂, der der Subklasse IgG1₁ angehört, eine L-Kette des κ-Typs hat und die folgenden Charakteristika besitzt:
(a) eine Affinitätskonstante von mehr als 10¹¹ M⁻¹;
(b) reagiert nicht mit Pankreasphospholipase A₂ vom Schwein oder Naja naja-Venom-Phospholipase A₂.

2. Hybridoma, das in der Lage ist, einen monoklonalen Antikörper gemäss Anspruch 1 zu produzieren.

3. Hybridoma, das in der Lage ist, einen monoklonalen Antikörper zu produzieren, der gegen humane Pankreasphospholipase A₂ spezifisch ist, oder ein monoklonaler Antikörper, der gegen humane Pankreasphospholipase A₂ spezifisch ist, welche in beiden Fällen erhältlich sind aus ECACC 87040802 oder ECACC 87040803.

4. Verfahren zur entweder (a) Herstellung eines Hybridoma, das in der Lage ist, einen monoklonalen Antikörper gemäss Anspruch 1 zu produzieren, oder (b) zur Herstellung dieses Antikörpers, umfassend die Fusionierung von Anti-humane Pankreasphospholipase A₂-Antikörper-produzierenden Zellen mit Myelomazellen und, falls gewünscht, Kultivierung des Hybridoma, um eine Antikörperproduktion zu ermöglichen.

5. Immunoassay, vorzugsweise ein Radioimmunoassay, für humane Pankreasphospholipase A₂, bei dem ein monoklonaler Antikörper gemäss Anspruch 1 als Immunreagens verwendet wird.

6. Verfahren zur in vitro-Diagnose oder Beobachtung von Abdominalleiden, umfassend die Bestimmung von humaner Pankreasphospholipase A₂ unter Verwendung eines Immunoassays gemäss Anspruch 5.

7. Immunoassay-Kit, umfassend einen monoklonalen Antikörper gemäss Anspruch 1, Instruktionen zur Ausführung eines Immunoassays gemäss Anspruch 5 und gegebenenfalls einen Marker, der die anschliessende Detektion eines Komplexes, der diesen Antikörper und humane Pankreasphospholipase A₂ umfasst, erlaubt.

## Revendications

1. Un anticorps monoclonal spécifique de la phospholipase pancréatique humaine A₂ qui est de la sous-classe IgG1₁, a une chaîne L du type α et a les caractéristiques suivantes:
a) une constante d'affinité supérieure à 10¹¹ M⁻¹;
b) ne réagit pas avec la phospholipase pancréatique A₂ du porc ou la phospholipase A₂ du venin de Naja naja.

2. Un hybridome capable de produire un anticorps monoclonal comme revendiqué dans la revendication 1.

3. Un hybridome capable de produire un anticorps monoclonal spécifique de la phospholipase pancréatique humaine A₂ ou un anticorps monoclonal spécifique de la phospholipase pancréatique humaine A₂, dans l'un ou l'autre cas pouvant être obtenu de ECACC 87040802 ou ECACC 87040803.

4. Un procédé soit a) pour la production d'un hybridome capable de produire un anticorps monoclonal comme cela est défini dans la revendication 1 soit b) pour la production dudit anticorps, comprenant la fusion de la phospholipase pancréatique anti-humaine A₂ produisant des cellules avec des cellules de myélome et, si on le souhaite, la culture de l'hybridome pour permettre la production d'anticorps.

5. Un essai immunologique, de préférence, un essai radioimmunologique, pour la phospholipase pancréatique humaine A₂ utilisant un anticorps monoclonal tel qu'il est défini dans la revendication 1 connue étant un réactif immulogique.

6. Un procédé de diagnostique in vitro ou de monitorage de l'état de l'abdomen, comprenant l'essai de la phospholipase pancréatique humaine A₂ en employant un essai immunologique comme cela a été défini dans la revendication 5.

7. Un nécessaire d'essai immunologique comprenant un anticorps monoclonal tel qu'il a été défini dans la revendication 1, des instructions pour réaliser un essai immunologique comme cela a été défini dans la revendication 5, et en option un marqueur permettant la détection ultérieure d'un complexe comprenant ledit anticorps et ladite phospholipase pancréatique humaine A₂.
